# EUROPEAN PATENT SPECIFICATION

(11) **EP 0 298 752 B1**
(45) Date of publication and mention of the grant of the patent: **02.02.1994**
(21) Application number: 88306238.2
(22) Date of filing: 07.07.1988
(51) Int. Cl.: C07D 213/82, C07D 213/89, C07D 401/12, A01N 47/36

(54) **Mercapto-substituted pyridine compounds and process for preparing the same**
Mercapto-substituierte Pyridine und Verfahren zu deren Herstellung
Pyridine mercapto-substituées et procédé pour leur préparation

(30) Priority: 10.07.1987 JP 172452/87; 24.02.1988 JP 41269/88; 26.03.1988 JP 72771/88
(43) Date of publication of application: 11.01.1989
(60) Divisional application: 92112827.8
(73) Proprietor: ISHIHARA SANGYO KAISHA, LTD., Nishi-ku Osaka (JP)
(72) Inventor: Haga, Takahiro c/o Ishihara Sangyo Kabushiki, Kusatsu-shi Shiga-ken (JP); Isogai, Tatsuo c/o Ishihara Sangyo Kabushiki, Kusatsu-shi Shiga-ken (JP); Tsujii, Yasuhiro c/o Ishihara Sangyo Kabushiki, Kusatsu-shi Shiga-ken (JP); Murai, Shigeo c/o Ishihara Sangyo Kabushiki, Kusatsu-shi Shiga-ken (JP); Jyonishi, Hisayoshi c/o Ishihara Sangyo Kabushiki, Kusatsu-shi Shiga-ken (JP); Sasaki, Hiroshi c/o Ishihara Sangyo Kabushiki, Kusatsu-shi Shiga-ken (JP); Kimura, Tokiya c/o Ishihara Sangyo Kabushiki, Kusatsu-shi Shiga-ken (JP)
(74) Representative: Pearce, Anthony Richmond

(56) References cited:
- EP-A- 0 232 067
- EP-A- 0 237 292
- DE-A- 3 342 538
- US-A- 3 759 932
- US-A- 4 435 206
- US-A- 4 518 776

## Description

The present invention relates to a novel mercapto-substituted pyridine compound and salts thereof, and to a process for preparing the same.

The mercapto-substituted pyridine compound of the present invention is characterized by having a dialkylaminocarbonyl group on the pyridine ring. Certain substituted pyridine compounds are disclosed in the respective specifications of U.S.P. 3,759,932; U.S.P. 4,435,206; U.S.P. 4,518,776 and U.S.P. 4,521,597. However, the above specifications fail to disclose the aforementioned mercapto-substituted pyridine compound.

US-A-3759932 discloses a method of preparing mercaptopyridines where a halosubstituted pyridine, which is optionally further substituted in the pyridine ring by C₁-C₄ alkyl or amino, is reacted with an alkali metal polysulfide.

The present invention provides a mercapto-substituted pyridine compound having the general formula (I): 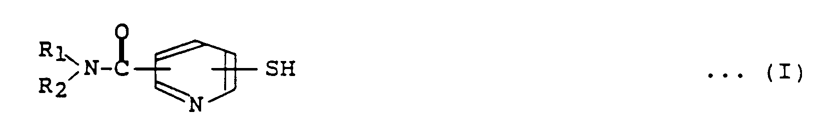
where each of R₁ and R₂ is a C₁-C₆ alkyl group;
or a salt thereof.

The alkyl group in R₁ and R₂ of the general formula (I) includes for example, a methyl group, ethyl group, propyl group, and butyl group.

The salt of the mercapto-substituted pyridine compound includes acid addition salts, alkali metal salts and the like, and specifically includes salts of the mercapto-substituted pyridine compounds of the general formula (I) with an alkali metal such as lithium, sodium, potassium or the like.

Among the mercapto-substituted pyridine compounds and salts thereof, a compound having the general formula (I'): 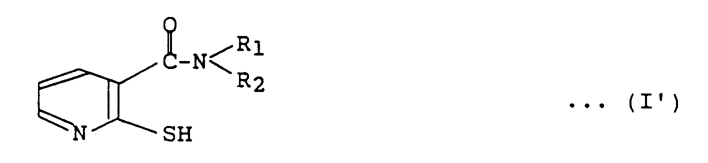
where R₁ and R₂ are as defined above, or a salt thereof, and further such a combination that R₁ is a methyl group and R₂ is a methyl group in the general formula (I'), is more preferred.

The mercapto-substituted pyridine compound of the present invention may be prepared, as shown by the following equation: 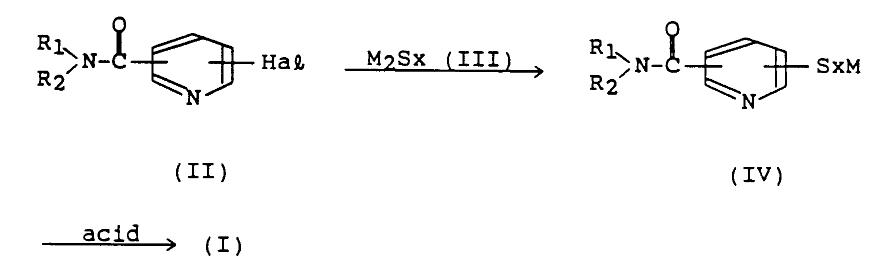
where M is an alkali metal, x is 2 to 8, Haℓ is a halogen atom, and R₁ and R₂ are as defined above, by a synthetic reaction of pyridinepolysulfide in which a halogeno-substituted pyridine compound having the general formula (II) is reacted with a polysulfide having the general formula (III) followed by an acid treatment.

### (Preparation of the halogeno-substituted pyridine compound)

The halogeno-substituted pyridine compound may be easily prepared, for example, by reacting thionyl chloride with a halogenopicolinic acid or halogenonicotinic acid or halogenoisonicotinic acid followed by reacting with an amine in a solvent such as methylene chloride. Among the halogeno-substituted pyridine compounds, such ones that the halogen atom located at 2, 4 or 6 position of the pyridine ring are desirable, and the halogen atom therein is desirably chlorine atom or bromine atom.

### (Synthetic reaction of pyridinepolysulfide)

The polysulfide used may include those obtained beforehand by reacting 1 to 7 moles, desirably 1 to 2 moles of sulfur with one mole of a mixture of an alkali metal hydroxide and a hydrosulfide thereof, or with one mole of an alkali metal sulfide according to the conventional process, and may also include those formed in the reaction system by reacting the above reactants in the presence of the halogeno-substituted pyridine compound of the general formula (II) to be directly used in situ. The alkali metal used in the alkali metal hydroxide, the hydrosulfide thereof or the sulfide thereof may include lithium, sodium and potassium, preferably sodium. The amount to be used of the hydroxide, hydrosulfide or sulfide is 0.75 to 5 moles, desirably 1 to 1.5 moles respectively per one mole of the halogeno-substituted pyridine compound. Normally water may be used as a solvent in the above reaction, but an organic solvent miscible with polysulfide and water may also be used. Examples of the organic solvent may include lower alcohol such as methanol, ethanol and propanol, polyalcohol such as ethylene glycol or propylene glycol, ethers such as tetrahydrofuran, an aprotic polar solvent such as dioxane or dimethylsulfoxide, ketones such as methyl ethyl ketone, nitriles such as acetonitrile. The amount of the solvent used is normally 10 to 1000% by weight, desirably 10 to 100% by weight based on the halogeno-substituted pyridine compound. Other reaction conditions of the above reaction may not be generally defined, but the reaction temperature is normally 0°C to reflux temperature, desirably 80 to 150°C, the reaction pressure is atmospheric pressure to several atoms, and the reaction time is generally 0.5 to 30 hours.

### (Acid treatment)

Since according to the above reaction, the mercapto-substituted pyridine compound is usually formed as an alkali metal salt of polysulfide, application of the conventional acid treatment to the reaction product results in liberating the intended mercapto-substituted pyridine compound, generating hydrogen sulfide gas, and in forming sulfur. The acid treatment is carried out, for example, by adding a non-oxidative mineral acid such as a concentrated hydrochloric acid or a dilute sulfuric acid to the reaction product in such an amount that the pH therein becomes 3 or lower, followed by subjecting to the conventional purification and separation procedure, resulting in making it possible to isolate the intended mercapto-substituted pyridine compound.

Typical examples of the mercapto-substituted pyridine compound having the general formula (I) and salts thereof are shown as follows:
- Compound No.1:: N,N-dimethyl-2-mercaptonicotinamide,
m.p. 214 - 215°C
- Compound No.2:: sodium salt of N,N-dimethyl-2-mercapto-nicotinamide,
m.p. 267 - 271°C
- Compound No.3:: N,N-diethyl-2-mercapto-nicotinamide,
m.p. 207 - 210°C
- Compound No.4:: sodium salt of N,N-diethyl-2-mercapto-nicotinamide
- Compound No.5:: 4-(N,N-dimethylaminocarbonyl)-2-mercapto-pyridine
the above mercapto-substituted pyridine compound or salt thereof to oxidation and halogenation reaction results in obtaining halogenosulfonyl-substituted pyridine compound.

The oxidation and halogenation process may include a process in which the mercapto-substituted pyridine compound or salt thereof is reacted with a halogenating agent such as chlorine gas, bromine or the like in the presence of water, an agueous hydrochloric acid solution, acetic acid or an aqueous acetic acid solution; a process in which the mercapto-substituted pyridine compound or salt thereof is reacted with hypochlorite or hypobromite in the presence of water or an aqueous hydrochloric acid solution the former being desirable.

In the above processes, an aprotic organic solvent may be used to make simple the post treatment of the reaction. Examples of the solvent used include benzene, hexane, toluene, xylene, methylene chloride, chloroform, carbon tetrachloride, ethylene dichloride, trichloroethylene, diethyl ether, ethyl acetate and the like. The amount of the solvent used is normally 50 to 2000% by weight based on the halogeno-substituted pyridine compound.

In the above processes, the amount of chlorine, bromine, hypochlorite, and hypobromite is in the range of from a theoretical amount for reaction to such an amount as to exceed slightly the theoretical amount based on the mercapto-substituted pyridine compound or salt thereof. Similarly thereto, the amount of water, the aqueous hydrochloric acid solution, acetic acid, the aqueous acetic acid solution or the like is 50 to 2000% by weight. The aqueous hydrochloric acid solution or the aqueous acetic acid solution may be used at a concentration of 1 to 30% by weight. In the above processes, other reaction conditions are not generally defined, but the reaction temperature is normally -20 to +50°C, desirably -10 to +10°C, and the reaction time is 0.1 to 5 hours. Application of the conventional purification and separation procedures to the reaction product results in making it possible to separate halogenosulfonyl-substituted pyridine compound.

Typical examples of the halogenosulfonyl-substituted pyridine compound are shown as follows.
- Compound No.A:: 2-chlorosulfonyl-N,N-dimethylnicotinamide, m.p. 114 - 117°C
- Compound No.B:: 2-bromosulfonyl-N,N-dimethylnicotinamide, m.p. 108 - 111°C
- Compound No.C:: 2-chlorosulfonyl-N,N-diethylnicotinamide, oily substance
- Compound No.D:: 2-bromosulfonyl-N,N-diethylnicotinamide
Reaction of the halogenosulfonyl-substituted pyridine compound with ammonia gas or ammonia water at -10 to +100°C leads to aminosulfonyl-substituted pyridine compound, a further reaction of which with 2-isocyanate-4,6-dimethoxypyrimidine or 2-chlorocarbonylamino-4,6-dimethoxy-pyrimidine at -10 to +100°C easily leads to N-[(4,6-dimethoxypyrimidin-2-yl) aminocarbonyl]-3-aminocarboxyl-2-pyridine sulfonamide compound. Application of the pyridine sulfonamide compound in an amount of 0.1 to 100 g per one are makes it possible to effectively control various kinds of harmful weeds and it has such high safety for corn as to be useful as a herbicide for the corn field.

Explanations are given on examples of the present invention as well as preparation examples of pyridine sulfonamide compound.

### Example 1

### (Synthetic reaction of pyridinepolysulfide)

A mixture of 25.0 g (0.1 mole) of 96% sodium sulfide nonahydrate, 10.4 g (0.325 mole) of sulfur and 56 mℓ of water is refluxed by heating. At the time when sulfur dissolves completely to form a homogeneous solution, 18.45 g (0.1 mole) of 2-chloro-N,N-dimethylnicotinamide is added, followed by refluxing by heating for 18 hours to form sodium salt of N,N-dimethyl-nicotinamide-2-polysulfide.

### (Acid treatment)

The above reaction product is allowed to cool down to room temperature, 15 mℓ of concentrated hydrochloric acid is carefully dropped (accompanying generation of hydrogen sulfide and precipitation of sulfur), and after the completion of dropping, agitation is carried out for 15 minutes. Sulfur is filtered off and washed with warm water, and the filtrate and washing liquor are confirmed to be evaporated to dryness under vacuum. The residue is repeatedly extracted with chloroform and dried over anhydrous solution sulfate, and chloroform is distilled off and the residual substance is purified with a silica gel column chromatography (developing solvent: methanol/chloroform = 1/9) to obtain 17.2 g of a yellow crystalline N,N-dimethyl-2-mercaptonicotinamide (m.p. 214-215°C).

### Example 2

### (Synthetic reaction of pyridinepolysulfide)

A mixture of 5.25 g (0.021 mole) of 96% sodium sulfide nonahydrate, 2.2 g (0.069 mole) of sulfur and 5 mℓ of water is refluxed by heating. At the time when the sulfur dissolves completely to form a homogeneous solution, 4.46 g (0.021 mole) of 2-chloro-N,N-diethylnicotinamide is added, followed by being refluxed by heating for 10 hours to form a sodium salt of N,N-diethylnicotinamide-2-polysulfide.

### (Acid treatment)

After the above reaction product is allowed to cool down to room temperature, 4 mℓ of concentrated hydrochloric acid is carefully dropped (accompanying generation of hydrogen sulfide and precipitation of sulfur). After the completion of dropping procedure, agitation is further carried out for 15 minutes. The aqueous phase is extracted with dichloromethane, followed by dry and distilling off the solvent under vacuum and purification of the residue with a silica gel column chromatography (developing solvent: methanol/chloroform = 1/19) to obtain 4.09 g of an yellow crystalline N,N-diethyl-2-mercaptonicotinamide (m.p. 207-210°C).

### Example 3

### (Synthetic reaction of pyridinepolysulfide)

A mixture of 24 g (0.3 mole) of sodium hydrosulfide of 70% purity, 9.6 g (0.3 mole) of sulfur, 12 g (0.3 mole) of sodium hydroxide and 15 mℓ of water is refluxed by heating. At the time when the sulfur dissolves completely to form a homogeneous solution, 55.4 g (0.3 mole) of 2-chloro-N,N-dimethylnicotinamide is added, followed by being refluxed (125°C) by heating for 2 hours to form a sodium salt of N,N-dimethylnicotinamide-2-polysulfide.

### (Acid treatment)

After the above reaction product is allowed to cool down to room temperature, 150 mℓ of water is added and about 30 mℓ of concentrated hydrochloric acid is dropped so as to adjust to pH 2, while hydrogen sulfide generates and sulfur precipitates. After the completion of dropping, agitation is carried out at 60 to 70°C for 30 minutes, followed by filtering off the sulfur while warming and by washing the residue with 150 mℓ of warm water to obtain filtrate and washing liquor which contain N,N-dimethyl-2-mercapto-nicotinamide.

### (Oxidation and bromination reaction)

The above mixture of filtrate and washing liquor are cooled with a mixture of sodium chloride and ice, 147 g (0.92 mole) of bromine is dropped with stirring at 5°C or lower, 100 mℓ of a cold water is then charged to be stirred, at -5°C for one hour. The reaction product is extracted with 700 mℓ of cold methylene chloride to obtain an extract liquor containing 2-bromosulfonyl-N,N-dimethylnicotinamide.

### Example 4

The oxidation and bromination reaction in Example 3 is varied as follows. Into 300 mℓ of water is suspended 54.6 g (0.3 mole) of N,N-dimethyl-2-mercaptonicotinamide to be cooled with agitation, and 144 g (0.9 mole) of bromine is dropped at -6°C to 0°C. After the completion of dropping above, purification procedure is carried out in the same manner as in Example 3 to obtain a methylene chloride solution containing 2-bromosulfonyl-N,N-dimethylnicotinamide. The methylene chloride solution is washed with 300 mℓ of ice water and 200 mℓ of cooled 1% aqueous solution of sodium thiosulfate, methylene chloride is distilled off at an inner temperature of 30°C or lower, followed by drying under vacuum to obtain 76 g of reaction product. The reaction product is dissolved in warm ethylene dichloride and is recrystallized with n-hexane to obtain 64 g (yield: 72.8%) of 2-bromosulfonyl-N,N-dimethylnicotinamide (m.p. 108-111°C).

### Example 5

### (Synthetic reaction of pyridinepolysulfide)

A mixture of 55.4 g of 2-chloro-N,N-dimethylnicotinamide, 24 g of sodium hydrosulfide of 70% purity, 9.6 g of sulfur, 12 g of sodium hydroxide and 15 mℓ of water is refluxed by heating with agitation for about 2 hours to form a sodium salt of N,N-dimethylnicotinamide-2-polysulfide.

### (Acid treatment)

To the above reaction product are added 150 mℓ of water and 30 mℓ of a 50% aqueous sulfuric acid solution to be stirred at 60 to 70°C for 30 minutes, and the precipitated sulfur is filtered off with warming. The sulfur is washed with 100 mℓ of warm water, and the filtrate and washing liquor are combined to obtain a solution containing N,N-dimethyl-2-mercaptonicotinamide.

### (Oxidation and bromination reaction)

The above solution is cooled down to 0°C or lower, 350 mℓ of methylene chloride is added, 144 g of bromine is dropped over about 20 minutes, and a methylene chloride layer is separated to obtain a methylene chloride solution of 2-bromosulfonyl-N,N-dimethylnicotinamide.

### Example 6

### (Synthetic reaction of pyridinepolysulfide)

A mixture of 26.4g (0.105 mole) of 96% sodium sulfide nonahydrate, 10.8g (0.338 mole) of sulfur and 56 mℓ of water is refluxed by heating. At the time when a homogeneous solution is formed, 18.45g (0.1 mole) of 2-chloro-N,N-dimethylnicotinamide is added, followed by refluxing by heating for 20 hours to form a sodium salt of N,N-dimethyl-nicotinamide-2-polysulfide.

### (Acid treatment)

The above reaction product is allowed to cool down to room temperature, 15 mℓ of concentrated hydrochloric acid is then carefully dropped. After the completion of dropping, stirring is carried out for 15 minutes to form N,N-dimethyl-2-mercaptonicotinamide.

### (Oxidation and chlorination reaction)

The resulting insoluble matter is filtered off and washed with warm water, the filtrate and washing liquor are combined, about 50 mℓ of dichloromethane is added to the combined filtrate and washing liquor to be ice-cooled, and chlorine gas is introduced therein. After confirming disappearance of N,N-dimethyl-2-mercaptonicotinamide, introduction of chlorine gas is stopped to form 2-chlorosulfonyl-N,N-dimethylnicotinamide (m.p. 114-117°C). The reaction mixture is charged into ice water, the dichloromethane layer is separated and collected, the collected dichloromethane layer is combined with one obtained by extracting the water layer with dichloromethane to be washed with water, followed by drying over anhydrous sodium sulfate and by cooling with ice water again.

### (Amidation reaction)

Ammonia gas is introduced into the resulting dichloromethane layer at 10°C or lower. At the time when the reaction mixture becomes weakly alkaline, introduction of ammonia gas is stopped. Dichloromethane is distilled off the reaction product, the remaining white crystals are washed with ethyl acetate followed by water, and dried to obtain 15.5 g of 2-aminosulfonyl-N,N-dimethylnicotinamide (m.p. 209-211°C).

### (Condensation)

A mixed solution containing 250 mg of 2-amino-4,6-dimethoxypyrimidine, 0.65 g of triethylamine and 2.5 g of ethyl acetate is dropped at 15°C into 6.3 g of an ethyl acetate solution of 20% phosgene to be reacted for one hour keeping the temperature at 15°C, followed by warming in an oil bath at 90°C to distill off excessive phosgene and ethyl acetate, adding a solution prepared by dissolving 300 mg of 2-aminosulfonyl-N,N-dimethylnicotinamide in 10 mℓ of acetonitrile, and by dropping 0.2 g of triethylamine to be reacted for one hour at room temperature.

After the completion of the reaction, the reaction product is charged into water, followed by acidifying with hydrochloric acid, filtering off deposited crystals, washing with water, and by drying to obtain 0.46 g of N-[(4,6-dimethoxypyrimidin-2-yl) aminocarbonyl]-3-dimethylaminocarbonyl-2-pyridinesulfonamide (m.p. 169-173°C).

### Example 7

A field soil is packed in a 1/1,500 are pot, and seeds of various kinds of plants are sowed thereon. When respective plants reach certain plant stages in leaf number respectively (corn : 3.2 leaf stage, wheat : 3.5 leaf stage, common cocklebur : 2.5 leaf stage, tall morningglory : 1.0 leaf stage, smartweed : 1.2 leaf stage, prickly sida : 1.0 leaf stage, slender amaranth : 0.5 leaf stage, barnyard grass: 2.0 leaf stage), a wettable powder of N-[(4,6-dimethoxypyrimidine-2-yl)aminocarbonyl]-3-dimethylaminocarbonyl-2-pyridinesulfonamide is weighed by such an amount as to be 1.25 (g/a) (as an amount of the active ingredient) to be diluted with SL of water per one are, followed by adding to the aqueous solution, an agricultural spreader by such an amount as to be 0.2% to be subjected to foliar application with a small-sized sprayer. Twenty four days after application, growth conditions of respective plants are observed visually and a degree of growth control is evaluated by ten grades (1 : the same as in non-application area to 10 : complete control). The results are shown below.

| Plants | Degree of Growth Control |
|---|---|
| Corn | 1 |
| Wheat | 8 |
| Common cocklebur | 10 |
| Tall morningglory | 8 |
| Prickly sida | 7 |
| Smartweed | 8 |
| Slender amaranth | 10 |
| Barnyard grass | 10 |

## Claims (Claims for the following Contracting State(s): AT, BE, CH, DE, FR, GB, GR, IT, LI, LU, NL, SE)

1. A mercapto-substituted pyridine compound having the general formula (I): 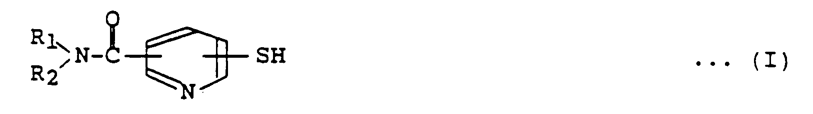 where each of R₁ and R₂ is a C₁-C₆ alkyl group; or a salt thereof.

2. A compound or salt thereof as claimed in claim 1, characterized in that said mercapto-substituted pyridine compound or salt thereof is a mercapto-substituted nicotinamide compound having the general formula (I'): 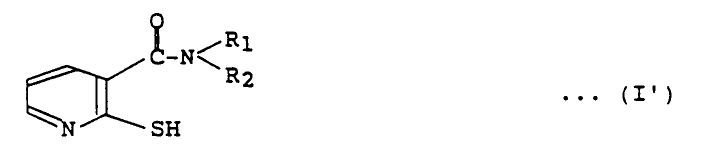 where R₁ and R₂ are as defined in claim 1;
or a salt thereof.

3. A compound or salt thereof as claimed in claim 2, characterized in that said mercapto-substituted nicotinamide compound is N,N-dimethyl-2-mercaptonicotinamide.

4. A process for preparing a mercapto-substituted pyridine compound or a salt thereof, which process comprises reacting a halogeno-substituted pyridine compound having the general formula (II): 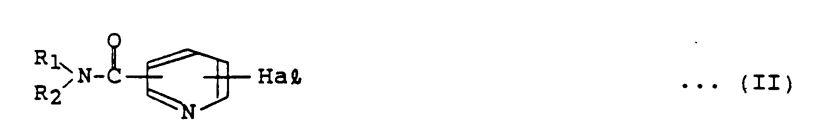 where each of R₁ and R₂ is a C₁-C₆ alkyl group, and Hal is a halogen atom, with a polysulfide having the formula: M₂Sₓ where M is an alkali metal and x is 2 to 8, followed by acid treatment to obtain a mercapto-substituted pyridine compound having the general formula (I): 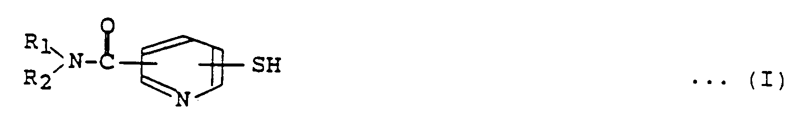 where R₁ and R₂ are as defined above; or a salt thereof.

5. A process as claimed in claim 4, characterized in that said mercapto-substituted pyridine compound is prepared by use of such a polysulfide that said alkali metal element is sodium and of a mineral acid as an acid treating agent.

6. The use of N,N-dimethyl-2-mercaptonicotinamide as an intermediate for manufacture of the herbicide N-[4,6-dimethoxypyrimidin-2-yl)aminocarbonyl]-3-dimethylaminocarbonyl-2-pyridinesulfonamide.

7. A polysulfide having the general formula (IV) 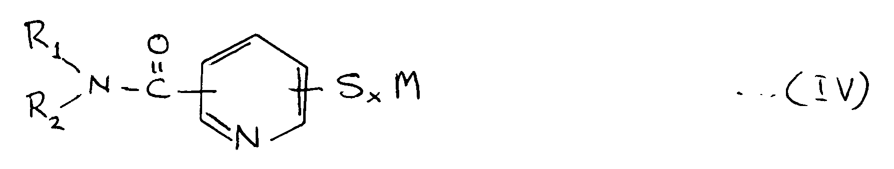 where each of R₁ and R₂ is a C₁-C₆ alkyl group, M is an alkali metal and x is 2 to 8.

8. A polysulfide as claimed in claim 7, which is a sodium salt of N,N-dimethylnicotinamide-2-polysulfide.

9. A process for producing a polysulfide having the general formula (IV) as defined in claim 7, which process comprises reacting a halogeno-substituted pyridine compound having the general formula (II): 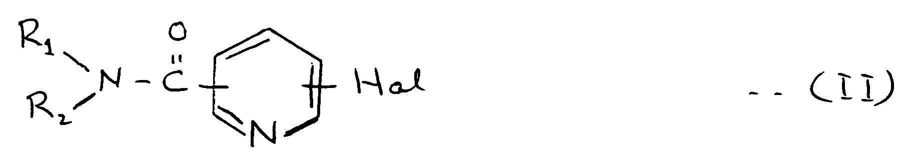 where each of R₁ and R₂ is a C₁-C₆ alkyl group, and Hal is a halogen atom, with a polysulfide having the formula: M₂Sₓ where M is an alkali metal and x is 2 to 8.

## Claims (Claims for the following Contracting State(s): ES)

1. A process for preparing a mercapto-substituted pyridine compound or a salt thereof, which process comprises reacting a halogeno-substituted pyridine compound having the general formula (II): 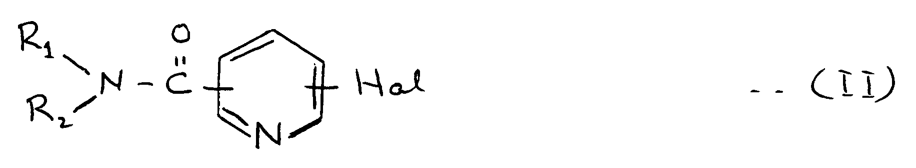 where each of R₁ and R₂ is a C₁-C₆ alkyl group, and Hal is a halogen atom, with a polysulfide having the formula: M₂Sₓ where M is an alkali metal and x is 2 to 8, followed by acid treatment to obtain a mercapto-substituted pyridine compound having the general formula (I): 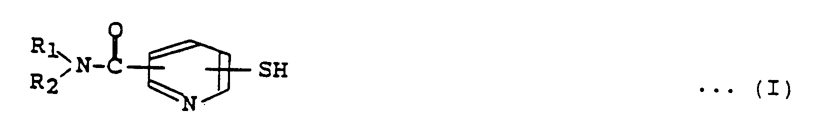 where R₁ and R₂ are as defined above; or a salt thereof.

2. A process as claimed in claim 1, characterized in that said mercapto-substituted pyridine compound or salt thereof is a mercapto-substituted nicotinamide compound having the general formula (I'): 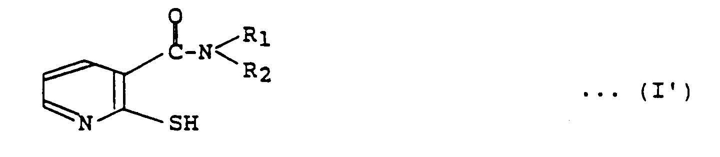 wherein R₁ and R₂ are as defined in claim 1; or a salt thereof.

3. A process as claimed in claim 2, characterised in that said mercapto-substituted nicotinamide compound is N,N-dimethyl-2-mercaptonicotinamide.

4. A process as claimed in any preceding claim, characterised in that said mercapto-substituted pyridine compound is prepared by use of such a polysulfide that said alkali metal element is sodium and of a mineral acid as an acid treating agent.

5. The use of N,N-dimethyl-2-mercaptonicotinamide as an intermediate for manufacture of the herbicide N-[4,6-dimethoxypyrimidin-2-yl)aminocarbonyl]-3-dimethylaminocarbonyl-2-pyridinesulfonamide.

6. A process for producing polysulfide having the general formula (IV) 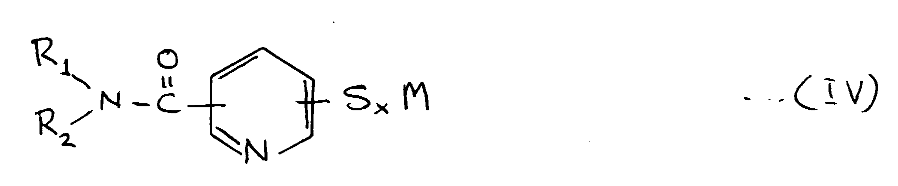 where each of R₁ and R₂ is a C₁-C₆ alkyl group, M is an alkali metal and x is 2 to 8, which process comprises reacting a halogeno-substituted pyridine having the general formula (II): 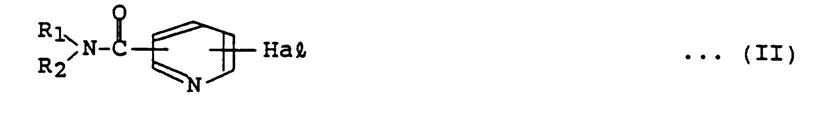 where each of R₁ and R₂ is a C₁-C₆ alkyl group, and Hal is a halogen atom, with a polysulfide having the formula: M₂Sₓ where M is an alkali metal and x is 2 to 8.

7. A process as claimed in claim 6, wherein the polysulfide is a sodium salt of N,N-dimethylnicotinamide-2-polysulfide.

## Patentansprüche (Patentansprüche für folgende(n) Vertragsstaat(en): AT, BE, CH, DE, FR, GB, GR, IT, LI, LU, NL, SE)

1. Mercapto-substituierte Pyridin-Verbindung der allgemeinen Formel (I): 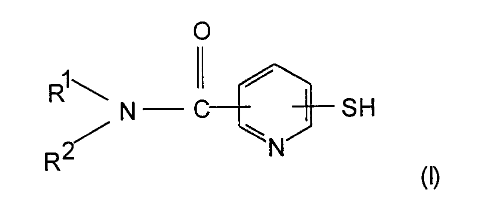 wobei sowohl R¹ wie auch R² eine C₁-C₆-Alkylgruppe ist; oder ein Salz davon.

2. Verbindung oder Salz nach Anspruch 1, dadurch **gekennzeichnet,** dass die mercaptosubstituierte Pyridin-Verbindung oder deren Salz eine mercaptosubstituierte Nicotinamid-Verbindung der allgemeinen Formel (I') ist: 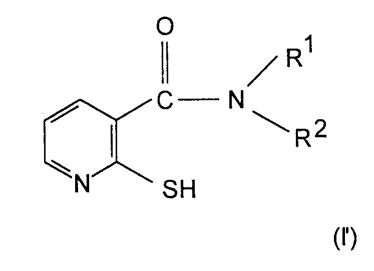 wobei R¹ und R² wie in Anspruch 1 definiert sind; oder ein Salz davon.

3. Verbindung oder Salz nach Anspruch 2, dadurch **gekennzeichnet,** dass die mercaptosubstituierte Nicotinamid-Verbindung N,N-Dimethyl-2-mercaptonicotinamid ist.

4. Verfahren zum Herstellen einer mercaptosubstituierten Pyridin-Verbindung oder ihres Salzes, umfassend: Umsetzen einer halogensubstituierten Pyridin-Verbindung der allgemeinen Formel (II) 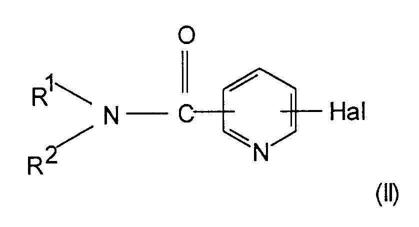 wobei sowohl R¹ wie auch R² eine C₁-C₆-Alkylgruppe ist und Hal ein Halogenatom bedeutet, mit einem Polysulfid der Formel M₂Sₓ, wobei M ein Alkalimetall ist und x 2 bis 8 ist, anschliessende Säurebehandlung unter Erhalt einer mercaptosubstituierten Pyridin-Verbindung der allgemeinen Formel (I) 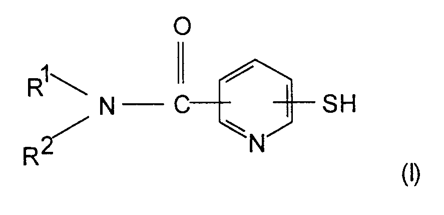 wobei R¹ und R² wie zuvor angegeben definiert sind, oder eines Salzes davon.

5. Verfahren gemäss Anspruch 4, dadurch **gekennzeichnet,** dass die mercaptosubstituierte Pyridin-Verbindung unter Verwendung eines derartigen Polysulfids, bei dem das Alkalimetallelement Natrium ist, und einer Mineralsäure als Säurebehandlungsagens hergestellt wird.

6. Verwendung von N,N-Dimethyl-2-mercaptonicotinamid als Zwischenverbindung für die Herstellung des Herbicids N-[(4,6-Dimethoxypyrimidin-2-yl)aminocarbonyl]-3-dimethylaminocarbonyl-2-pyridinsulfonamid.

7. Polysulfid der allgemeinen Formel (IV): 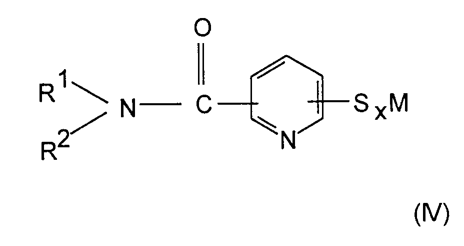 wobei sowohl R¹ wie auch R² eine C₁-C₆-Alkylgruppe ist, M ein Alkalimetall ist und x 2 bis 8 ist.

8. Polysulfid nach Anspruch 7, welches ein Natriumsalz von N,N-Dimethylnicotinamid-2-polysulfid ist.

9. Verfahren zum Herstellen eines Polysulfids der allgemeinen Formel (IV) nach Anspruch 7, umfassend Umsetzen einer halogensubstituierten Pyridin-Verbindung der allgemeinen Formel (II): 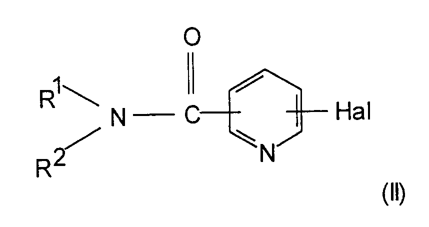 wobei sowohl R¹ wie auch R² eine C₁-C₆-Alkylgruppe ist und Hal ein Halogenatom ist, mit einem Polysulfid der Formel: M₂Sₓ, wobei M ein Alkalimetall ist und x 2 bis 8 ist.

## Patentansprüche (Patentansprüche für folgende(n) Vertragsstaat(en): ES)

1. Verfahren zum Herstellen einer mercaptosubstituierten Pyridin-Verbindung oder eines Salzes davon, umfassend Umsetzen einer halogensubstituierten Pyridin-Verbindung der allgemeinen Formel (II) 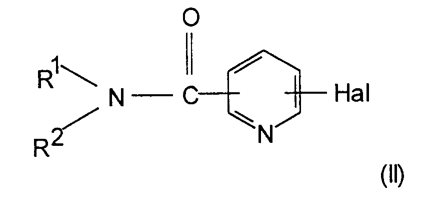 wobei sowohl R¹ wie auch R² eine C₁-C₆-Alkylgruppe ist und Hal ein Halogenatom bedeutet, mit einem Polysulfid der Formel M₂Sₓ, wobei M ein Alkalimetall ist und x 2 bis 8 ist, anschliessende Säurebehandlung unter Erhalt einer mercaptosubstituierten Pyridin-Verbindung der allgemeinen Formel (I) 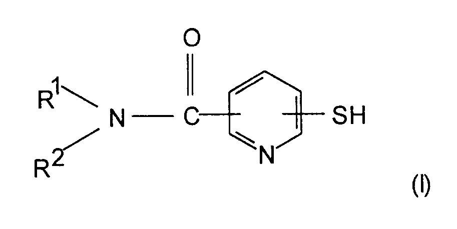 wobei R¹ und R² wie zuvor angegeben definiert sind, oder eines Salzes davon.

2. Verfahren nach Anspruch 1, dadurch **gekennzeichnet,** dass die mercaptosubstituierte Pyridin-Verbindung oder deren Salz eine mercaptosubstituierte Nicotinamid-Verbindung der allgemeinen Formel (I') 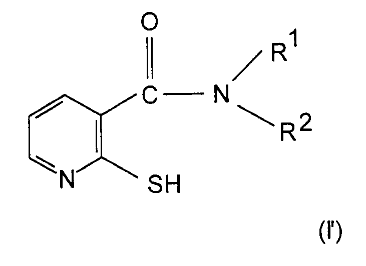 wobei R¹ und R² wie in Anspruch 1 definiert sind, oder ein Salz davon ist.

3. Verfahren nach Anspruch 2, dadurch **gekennzeichnet,** dass die mercaptosubstituierte Nicotinamid-Verbindung N,N-Dimethyl-2-mercaptonicotinamid ist.

4. Verfahren nach einem der vorhergehenden Ansprüche, dadurch **gekennzeichnet,** dass die mercaptosubstituierte Pyridin-Verbindung unter Verwendung eines derartigen Polysulfids, bei dem das Alkalimetallelement Natrium ist, und einer Mineralsäure als Säurebehandlungsmittel hergestellt wird.

5. Verwendung von N,N-Dimethyl-2-mercaptonicotinamid als Zwischenverbindung zur Herstellung des Herbicids N-[(4,6-Dimethoxypyrimidin-2-yl)aminocarbonyl]-3-dimethylaminocarbonyl-2-pyridinsulfonamid.

6. Verfahren zum Herstellen von Polysulfid der allgemeinen Formel (IV): 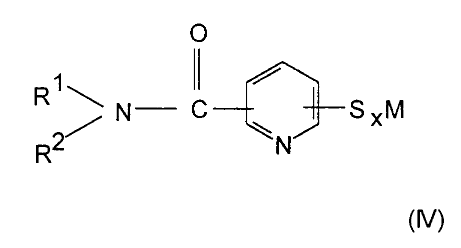 wobei sowohl R¹ wie auch R² eine C₁-C₆-Alkylgruppe ist, M ein Alkalimetall ist und x 2 bis 8 ist, umfassend Umsetzen eines halogensubstituierten Pyridins der allgemeinen Formel (II) 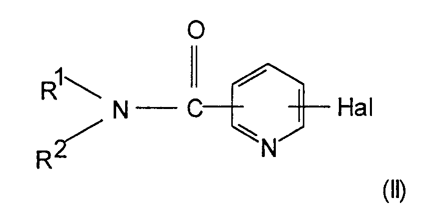 wobei sowohl R¹ wie auch R² eine C₁-C₆-Alkylgruppe ist und Hal ein Halogenatom ist, mit einem Polysulfid der Formel: M₂Sₓ, wobei M ein Alkalimetall ist und x 2 bis 8 ist.

7. Verfahren nach Anspruch 6, wobei das Polysulfid ein Natriumsalz von N,N-Dimethylnicotinamid-2-polysulfid ist.

## Revendications (Revendications pour l'(les) Etat(s) contractant(s) suivant(s): AT, BE, CH, DE, FR, GB, GR, IT, LI, LU, NL, SE)

1. Composé dérivé de pyridine mercapto-substituée de formule générale (I) : 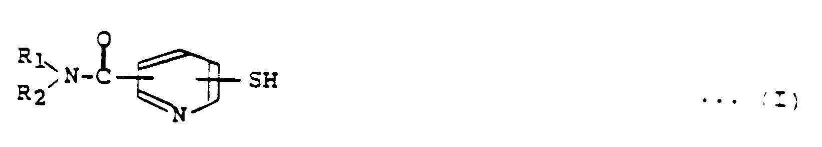 dans laquelle chacun des groupes R₁ et R₂ représente un groupe alkyle en C₁-C₆ ; ou un sel de celui-ci.

2. Composé ou un sel de celui-ci selon la revendication 1, caractérisé en ce que ce composé dérivé de pyridine mercapto-substituée ou son sel, est un composé dérivé de nicotinamide mercapto-substitué de formule générale (I') : 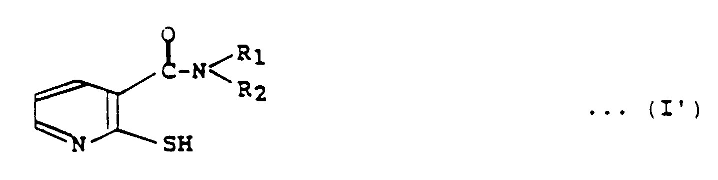 dans laquelle R₁ et R₂ sont tels que définis dans la revendication 1 ; ou un sel de celui-ci.

3. Composé ou sel de celui-ci selon la revendication 2, caractérisé en ce que le composé dérivé de nicotinamide mercapto-substitué, est le N,N-diméthyl-2-mercaptonicotinamide.

4. Procédé de préparation d'un composé dérivé de pyridine mercapto-substituée ou d'un sel de celui-ci, selon lequel on fait réagir un composé dérivé de pyridine halogéno-substituée de formule générale (II) : 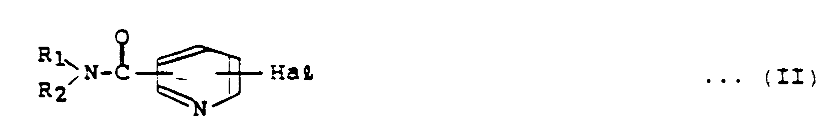 dans laquelle chacun des groupes R₁ et R₂ représente un groupe alkyle en C₁-C₆, et Hal représente un atome d'halogène, avec un polysulfure de formule M₂Sₓ dans laquelle M représente un métal alcalin et x varie de 2 à 8, puis on effectue un traitement acide pour obtenir un composé dérivé de pyridine mercapto-substituée de formule générale (I) : 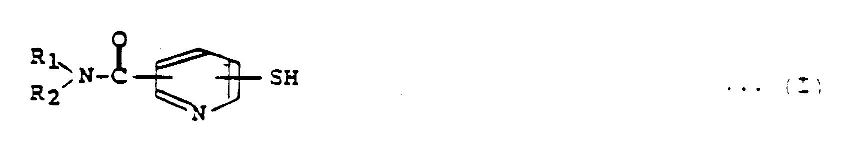 dans laquelle R₁ et R₂ sont tels que définis ci-dessus ; ou un sel de celui-ci.

5. Procédé selon la revendication 4, caractérisé en ce que le composé dérivé de pyridine mercapto-substituée, est préparé en employant un polysulfure tel que l'élément de métal alcalin soit le sodium, et un acide minéral comme agent de traitement acide.

6. Utilisation du N,N-diméthyl-2-mercaptonicotinamide comme intermédiaire pour la préparation du N-[4,6-diméthoxypyrimidin-2-yl)aminocarbonyl]-3-diméthylaminocarbonyl-2- pyridinesulfonamide herbicide.

7. Polysulfure de formule générale (IV) 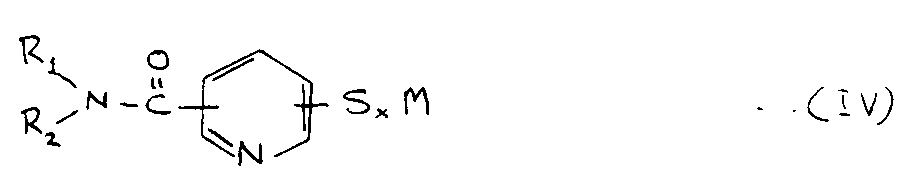 dans laquelle chacun des groupes R₁ et R₂ représente un groupe alkyle en C₁-C₆, M représente un métal alcalin et x varie de 2 à 8.

8. Polysulfure selon la revendication 7, qui est un sel de sodium de N,N-diméthylnicotinamide-2-polysulfure.

9. Procédé de préparation d'un polysulfure de formule générale (IV) selon la revendication 7, selon lequel on fait réagir un composé dérivé de pyridine halogéno-substituée de formule générale (II) : 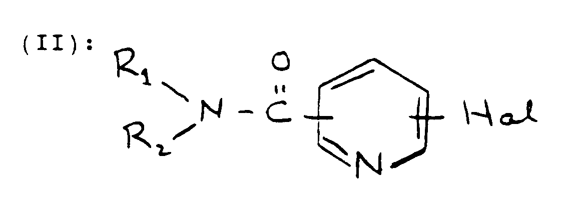 dans laquelle chacun des groupes R₁ et R₂ représente un groupe alkyle en C₁-C₆, et Hal représente un atome d'halogène, avec un polysulfure de formule M₂Sₓ dans laquelle M représente un métal alcalin et x varie de 2 à 8.

## Revendications (Revendications pour l'(les) Etat(s) contractant(s) suivant(s): ES)

1. Procédé de préparation d'un composé dérivé de pyridine mercapto-substituée ou d'un sel de celui-ci, selon lequel on fait réagir un composé dérivé de pyridine halogéno-substituée de formule générale (II) : 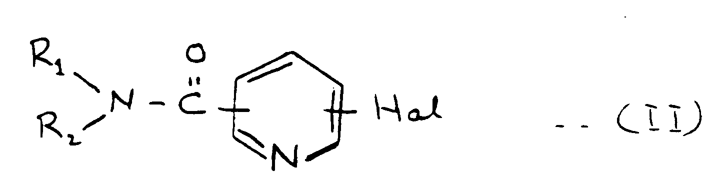 dans laquelle chacun des groupes R₁ et R₂ représente un groupe alkyle en C₁-C₆, et Hal représente un atome d'halogène, avec un polysulfure de formule M₂Sₓ dans laquelle M représente un métal alcalin, et x varie de 2 à 8, puis on effectue un traitement acide pour obtenir un composé dérivé de pyridine mercapto-substituée de formule générale (I) : 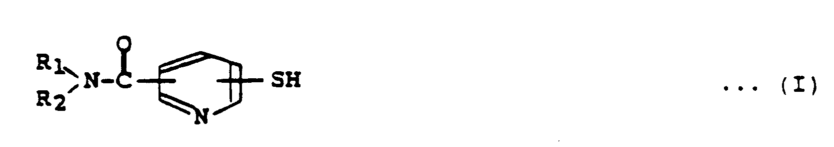 dans laquelle R₁ et R₂ sont tels que définis ci-dessus ; ou un sel de celui-ci.

2. Procédé selon la revendication 1, caractérisé en ce que ce composé dérivé de pyridine mercapto-substituée ou son sel, est un composé dérivé de nicotinamide mercapto-substitué de formule générale (I') : 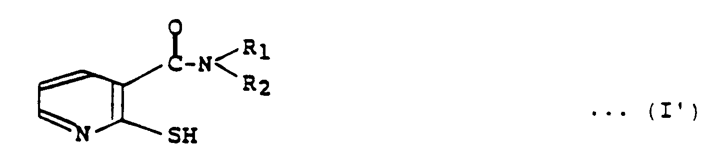 dans laquelle R₁ et R₂ sont tels que définis dans la revendication 1 ; ou un sel de celui-ci.

3. Procédé selon la revendication 2, caractérisé en ce que le composé dérivé de nicotinamide mercapto-substitué, est le N,N-diméthyl-2-mercaptonicotinamide.

4. Procédé selon l'une quelconque des revendications précédentes, caractérisé en ce que le composé dérivé de pyridine mercapto-substituée, est préparé en employant un polysulfure tel que l'élément de métal alcalin est le sodium, et un acide minéral comme agent de traitement acide.

5. Utilisation du N,N-diméthyl-2-mercaptonicotinamide comme intermédiaire pour la préparation du N-[4,6-diméthoxypyrimidin-2-yl)aminocarbonyl]-3-diméthylaminocarbonyl-2-pyridinesulfonamide herbicide.

6. Procédé de préparation d'un polysulfure de formule générale (IV) : 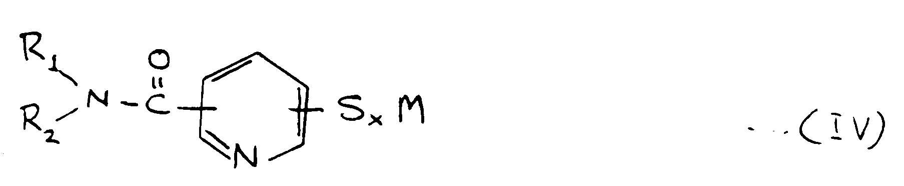 dans laquelle chacun des groupes R₁ et R₂ représente un groupe alkyle en C₁-C₆, M représente un métal alcalin et x varie de 2 à 8, selon lequel on fait réagir une pyridine halogéno-substituée de formule générale (II) : 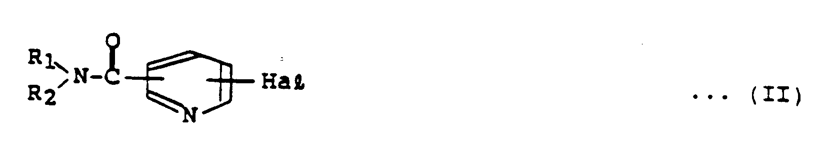 dans laquelle chacun des groupes R₁ et R₂ représente un groupe alkyle en C₁-C₆, et Hal représente un atome d'halogène, avec un polysulfure de formule M₂Sₓ dans laquelle M représente un métal alcalin et x varie de 2 à 8.

7. Procédé selon la revendication 6, dans lequel le polysulfure est un sel de sodium de N,N-diméthylnicotinamide-2-polysulfure.
